# EUROPEAN PATENT APPLICATION

(11) **EP 4 227 397 A1**
(43) Date of publication of application: **16.08.2023**
(21) Application number: 21880468.0
(22) Date of filing: 12.10.2021
(51) Int. Cl.: C12M 1/12, C12M 1/00, C12N 5/00, C12N 5/077, C12N 11/10, C12N 11/12

(54) **MULTI-LAYER FILM COMPRISING NUTRIENT MATERIAL FOR CELL CULTURE, METHOD FOR PREPARING SAME, AND USE OF SAME**

(30) Priority: 12.10.2020 KR 20200131409
(71) Applicant: CJ Cheiljedang Corporation, Seoul 04560 (KR)
(72) Inventor: LEE, Hyeong Rae, Seoul 04560 (KR); LEE, Seung Yeon, Seoul 04560 (KR); BAEK, Seung Jin, Seoul 04560 (KR); HONG, Jin Kee, Seoul 03722 (KR); PARK, So Hyeon, Seoul 03722 (KR)
(74) Representative: Cabinet Laurent & Charras
(86) International application number: PCT/KR2021/014023
(87) International publication number: WO 2022/080821

(57) **Abstract**

The present application relates to a multi-layer film comprising a nutrient material for cell culture, a method for preparing same, and the use of same. The multi-layer film of the present application can be utilized for cell culture for preparing cultured meat.

## Description

### TECHNICAL FIELD

The present application relates to a multi-layer film including a nutrient material for cell culture, a method for preparing same, and a use of same.

### BACKGROUND ART

Cultured meat is artificial meat prepared through mass culture of cells without slaughter of livestock, and is prepared through an ethical and eco-friendly process, unlike actual meat that requires a process of breeding and slaughtering livestocks and a use of antibiotics. The process for preparing cultured meat is performed through the following process: First, muscle satellite cells are isolated from livestock, and then the isolated cells are mass-proliferated in a culture medium containing amino acids, vitamins, sugars, inorganic salts, minerals, and various growth factors in a bioreactor. The prepared cells are transferred to a specific support or differentiated into muscle tissues in the differentiated culture medium through self-assembly between cells.

Although many companies around the world are challenging the cultured meat industry, it is difficult to commercialize the cultured meat due to the complex production process and high production costs. Since the cost of the culture medium used in the mass proliferation process of muscle cells isolated from livestock accounts for at least 70% of the total production cost of cultured meat, research for reducing the cost of the culture medium is an indispensable factor. The conventional culture used for cell proliferation includes fetal bovine serum (FBS) containing various growth factors, cytokines, proteins, and the like essential for cell growth. However, since the use of animal-derived components is not suitable for the original purpose of cultured meat, there is a demand for developing a technology capable of excluding or reducing FBS.

### [PRIOR ART DOCUMENTS]

Molecular Pharmaceutics 2017, 14, 3322-3330.

Korean Patent Laid-open Publication No. 10-2014-0100671

### DISCLOSURE OF THE INVENTION

### TECHNICAL PROBLEM

The present inventors have made efforts to develop a nutrient delivery platform capable of replacing or reducing animal-derived serum during cell culture for preparing cultured meat. As a result, the present inventors have proved experimentally that a cell proliferation effect superior to that of the serum-using culture medium can be achieved through the release of the nutrient materials by preparing a multi-layer film having pores from a polymer-derived material, incorporating nutrient materials in the pores of the prepared multi-layer film, and applying the resultant film in a cell culture medium, thereby completing the present application.

Therefore, an aspect of the present application provides a multi-layer film for delivering nutrient materials during cell culture.

In addition, another aspect of the present application provides a composition for cell culture including a multi-layer film.

In addition, further another aspect of the present application provides an apparatus for cell culture including a multi-layer film.

In addition, still another aspect of the present application provides a method for preparing cultured meat, the method including bringing a multi-layer film into contact with a cell culture medium containing cells.

In addition, yet another aspect of the present application provides a method for reducing serum components in a cell culture medium, the method including bringing a multi-layer film into contact with a cell culture medium containing cells.

In addition, still yet another aspect of the present application provides a method for preparing a multi-layer film for delivering nutrient materials during cell culture.

### TECHNICAL SOLUTION

In order to accomplish the object of the invention,
according to an aspect of the present application, there is provided a multi-layer film for cell culture including a substrate, and a multi-layer structure formed on the substrate.

According to another aspect of the present application, there is provided a composition for cell culture including the multi-layer film.

According to further another aspect of the present application, there is provided an apparatus for cell culture including the multi-layer film.

According to still another aspect of the present application, there is provided a method for preparing cultured meat, the method including bringing a multi-layer film into contact with a cell culture medium containing cells.

According to yet another aspect of the present application, there is provided a method for reducing serum components in a cell culture medium, the method including bringing a multi-layer film into contact with a cell culture medium containing cells.

According to still yet another aspect of the present application, there is provided a method for preparing a multi-layer film for cell culture.

Hereinafter, the present application will be described in detail.

According to an aspect, the present application provides a multi-layer film for cell culture including: (a) a substrate; and (b) a multi-layer structure which is formed by alternately stacking, on the substrate, a cationic material having an amine group in the side chain thereof, and an anionic material having a carboxyl group in the side chain thereof, and contains pores, and in which the cationic material and the anionic material are cross-linked to each other.

### Substrate

The substrate which may be used in the present application may be used without limitation in the case where the substrate has supporting characteristics and at the same time, an anionic material having a carboxyl group in the side chain thereof or a cationic material having an amine group in the side chain thereof can be bonded to be stacked on the substrate.

Materials that may be used as the substrate may be polymers, plastics (e.g., polystyrene, polyethylene, polypropylene, etc.), metals, metal oxides, silicone, ceramics, Si/SiO2 wafers, glass, carbon, carbon nanotubes, or biodegradable polymers, but are not limited thereto.

The substrate may be surface-treated to facilitate bonding with a cationic material or an anionic material, which will be described later.

The shape of the substrate is not particularly limited, but may be in the form of an incubator, a culture dish, a plate, a flask, a chamber slide, a tube, a cell factory, a roller bottle, a spinner flask, hollow fibers, a micro-carrier, beads, a fragment, and the like, considering that the multi-layer film of the present application is applied to cell culture.

### Multi-layer Structure

The multi-layer structure herein is formed by alternately stacking an anionic material having a carboxyl group in the side chain thereof and a cationic material having an amine group in the side chain thereof on the substrate.

The anionic material may be attached to the surface of the positively charged substrate, and the cationic material may be attached to the surface of the negatively charged substrate. Accordingly, when the substrate is negatively charged, the cationic material may form a first layer, and when the substrate is positively charged, the anionic material may form a first layer.

In the present application, the cationic material having an amine group in the side chain thereof may be bonded to the substrate to form a first layer.

In an embodiment, the cationic material having an amine group in the side chain thereof may be dissolved in a solution to be contact with the surface of the substrate, thereby forming the first layer. The degree of ionization of the cationic material, that is, the degree of protonation may be controlled by adjusting the pH of the solution to a predetermined range. When the pH of the solution is adjusted to a range of 3-5, at least 50%, at least 60%, at least 70%, at least 80%, or at least 90% of the amine group of the cationic material may be protonated. The protonated amine group (-NH3+) may be bonded to a deprotonated carboxyl group (-COO⁻) of the anionic material having a carboxyl group in the side chain thereof by electrostatic attraction. A multi-layer structure may be formed by electrostatic attraction between the cationic material and the anionic material.

The cationic material having an amine group in the side chain thereof may be any one selected from the group consisting of chitosan, gelatin, collagen, fibrinogen, silk fibroin, casein, elastin, laminin, fibronectin, polydopamine, polyethyleneimine, poly-l-lysine, poly(vinylamine)hydrochloride, and poly(amino acid).

In the present application, the cationic material having an amine group in the side chain thereof may be chitosan.

In the present application, the anionic material having a carboxyl group in the side chain thereof may be bonded on the first layer to form a second layer.

In an embodiment, the anionic material having a carboxyl group in the side chain thereof may be dissolved in a solution to be contact with the surface of the first layer of the cationic material to form a second layer.

The degree of ionization of the anionic material, that is, the degree of deprotonation may be controlled by adjusting the pH of the solution to a predetermined range. When the pH of the solution is adjusted to a range of 3-5, at least 50%, at least 60%, at least 70%, at least 80%, or at least 90% of the carboxyl group may be deprotonated (-COO-).

As described above, the deprotonated carboxyl group (-COO-) of the anionic material may be bonded to the protonated amine group (-NH3+) of the cationic material by electrostatic attraction, and the layer of the anionic material and the layer of the cationic material may be alternately stacked by the electrostatic attraction.

The anionic material having a carboxyl group in the side chain thereof may be, for example, any one selected from the group consisting of hyaluronic acid, poly-l-lactic acid, alginic acid, dextran sulfate, lignin, tannic acid, chondroitin sulfate, cellulose-based polymers, fucoidan, and heparin, and the cellulose-based polymer may be carboxymethylcellulose.

In the present application, the anionic material having a carboxyl group in the side chain thereof may be carboxymethylcellulose.

In the present application, when the cationic material having an amine group in the side chain thereof is chitosan, the anionic material having a carboxyl group in the side chain thereof may be carboxymethylcellulose.

When a process of stacking the first layer of the cationic material and the second layer of the anionic material is referred to as one cycle of stack process, the one cycle of stack process may be repeated twice or more to form a multi-layer structure.

Meanwhile, when the substrate is positively charged, an anionic material may be stacked as a first layer on the substrate, a cationic material may be coated on the surface of the first layer to be stacked as a second layer, and in a similar manner, when a process of stacking the first layer of the anionic material and the second layer of the cationic material is referred to as one cycle of stack process, the one cycle of stack process may be repeated twice or more to form a multi-layer structure.

In the present application, a layer including the first layer of the cationic material and the second layer of the anionic material, or the first layer of the anionic material and the second layer of the cationic material may be referred to as "1 bilayer (BL)" as one unit.

The thickness of the multi-layer structure may be controlled by adjusting the number of repetitions of the 1 BL stack process, and a person skilled in the art may appropriately adjust the number of repetitions of the stack process according to application conditions of the multi-layer film.

In the present application, one cycle of stack process forming 1 BL may be performed, for example, in the range of 10-70 times, 12-68 times, 14-66 times, 16-64 times, 18-62 times, 20-60 times, 22-58 times, 24-56 times, 26-54 times, 28-52 times, 30-50 times, 32-48 times, 34-46 times, or 36-44 times.

In the present application, the thickness of the multi-layer structure may also be expressed as the number of the BL, and for example, when the stack process is repeated 10-70 times, a multi-layer structure having a thickness of 10-70 BLs may be formed.

In an embodiment, the thickness of the multi-layer structure may be 10-70 BLs, 12-68 BLs, 14-66 BLs, 16-64 BLs, 18-62 BLs, 20-60 BLs, 22-58 BLs, 24-56 BLs, 26-54 BLs, 28-52 BLs, 30-50 BLs, 32-48 BLs, 34-46 BLs, or 36-44 BLs.

In the present application, the cationic material and the anionic material may be cross-linked to each other.

In the present application, the cationic material and the anionic material forming the multi-layer structure can form an amide bond by reacting a non-protonated amine group (-NH2) and a non-deprotonated free carboxyl group (-COOH), in each of the cationic and anionic materials, with each other, and the cationic material layer and the anionic material layer can be cross-linked by the amide bond.

The cross-linking may be performed in the presence of a cross-linking agent, and for example, the cross-linking may be performed by sequentially supporting a first cross-linking agent including 1-ethyl-3-(3-(dimethylamino)propyl)carbodiimide hydrochloride (EDC) and N-hydroxysulfosuccinimide (NHS), and a second cross-linking agent including glutaraldehyde.

Pores may be formed in the multi-layer structure by the cross-linking.

The size, roughness or porosity of pores formed in the multi-layer structure may be controlled by adjusting the concentration of the cross-linking agent and the reaction time of the cross-linking reaction during the cross-linking.

In addition, the porosity of pores may be controlled by adjusting not only the cross-linking reaction but also the thickness of the multi-layer structure.

In an embodiment, the multi-layer structure after the cross-linking may have a porosity in a range of 10-26%, 11-25%, 12-24%, 13-23%, 14-22%, 15-21%, or 16-20%. In an embodiment, the multi-layer structure after the cross-linking may have a degree of cross-linking of 50-80%, 52-78%, 54-76%, 56-74%, 58-72%, or 60-70%.

In an embodiment, the multi-layer structure after the cross-linking may have a degree of degradation of 10% or less in the physiological artificial saliva environment. This may maintain the effect of the multi-layer film for a long period of time in the process for preparing cultured meat.

In an embodiment, for the pore size of the multi-layer structure, the average size of the pores on the surface of the multi-layer structure is greater than the average size of the pores inside the multi-layer structure.

### Nutrient Materials

In the present application, nutrient materials for cell culture may be incorporated into the pores of the multi-layer structure. Accordingly, the multi-layer film for cell culture of the present application may further include the nutrient materials for cell culture incorporated in the multi-layer structure.

The incorporation of the nutrient materials into the pores may be performed, for example, by bringing the solution containing nutrient materials into contact with the multi-layer structure.

Since the nutrient materials for cell culture are incorporated into the pores of the multi-layer structure, the nutrient materials may have a molecular weight of 30 kDa or less in consideration of the size of the pores.

The nutrient materials for cell culture include hormones, growth factors, amino acids, or vitamins, and may be, for example, C-phycocyanin; growth factors selected from the group consisting of IGF-1, BMP-2, and FGF-1; amino acids; or ascorbic acid, but are not limited thereto.

The nutrient materials may be sufficiently incorporated into the pores on the surface of the multi-layer structure as well as the pores inside the multi-layer structure.

### Capping Layer

In an embodiment, a capping layer may be further formed on the above-described multi-layer structure. The capping layer may be formed on the outermost layer of the multi-layer structure.

The capping layer may be formed, for example, by bringing a solution containing a material capable of constituting the capping layer into contact with the multi-layer structure in which nutrient materials are incorporated.

The capping layer stabilizes the nutrient materials for cell culture incorporated into the pores, inhibits decomposition, and inhibits burst release.

The capping layer may be formed of polysaccharides, which may be, for example, selected from the group consisting of agarose, carageenan, fucoidan, laminaran, cellulose, hemicellulose, and lignan.

In the present application, when the nutrient material for cell culture is C-phycocyanin, the capping layer may be a capping layer of agarose.

In the present application, the thickness of the multi-layer film may be 0.1-10 um, more specifically 0.2-9 um, 0.3-8.5 um, 0.5-8.5 um, 0.8-8.5 µm, 1.0-8.0 µm, 1.2-8.0 um, 2.0-8.0 µm, 2.5-7.5 µm, 2.5-7.0 um, 2.5-6.5 µm, 2.7-6.0 um, or 3.0-6.0 um.

In an embodiment, the multi-layer film of the present application may be used for cell culture purposes.

According to another aspect, the present application provides a composition for cell culture, the composition containing the above-described multi-layer film and serum components.

In an embodiment, the serum component content may be less than 10 wt% in the composition for cell culture, and specifically less than 9 wt%, less than 8 wt%, less than 7 wt%, less than 6 wt%, less than 5 wt%, or less than 4 wt%.

The lower limit of the serum component content may be 0.001 wt%, 0.01 wt%, or 0.1 wt% in the composition for cell culture. In an embodiment, the composition for cell culture may be, for example, a medium for cell culture containing the above-described multi-layer film.

In an embodiment, the serum component content may be less than 10 wt%, more specifically less than 9 wt%, less than 8 wt%, less than 7 wt%, less than 6 wt%, less than 5 wt%, or less than 4 wt% in the medium for cell culture.

The lower limit of the serum component content may be 0.001 wt%, 0.01 wt%, or 0.1 wt% in the medium for cell culture.

In another embodiment, the serum component may be fetal bovine serum, and the fetal bovine serum content may be less than 10 wt%, more specifically less than 9 wt%, less than 8 wt%, less than 7 wt%, less than 6 wt%, less than 5 wt%, and less than 4 wt% in the medium for cell culture, and the lower limit of the fetal bovine serum content may be 0.001 wt%, 0.01 wt%, or 0.1 wt% in the medium for cell culture.

Even through the composition of the present application contains the serum component having a content lower than 10 wt%, which is the content of serum components (e.g., fetal bovine serum) contained in a conventional cell culture medium, when the composition contains the above-described multi-layer film of the present application, cell proliferation, more specifically, equal or higher level of proliferation in muscle cell proliferation, is possible.

In another embodiment, the cells may be cells for preparing cultured meat, and specifically, may be animal-derived muscle cells, and the muscle cells may include myoblast, muscle satellite cells, myotube cells, myotubes, myofibers, or mature myofibers.

According to further another aspect, the present application provides an apparatus for cell culture including the above-described multi-layer film.

In an embodiment, the apparatus for cell culture may be a cell culture dish provided with a multi-layer film, a cell culture plate, a cell culture container, or a cell incubator, but is not limited thereto. In another embodiment, the cells may be cells for preparing cultured meat, and specifically, may be animal-derived muscle cells, and the muscle cells may include myoblast, muscle satellite cells, myotube cells, myotubes, myofibers, or mature myofibers.

According to still another aspect, the present application provides a method for preparing cultured meat, the method including bringing the above-described multi-layer film into contact with a cell culture solution containing cells.

In an embodiment, the cells may be cells for preparing cultured meat, and specifically, may be animal-derived muscle cells, and the muscle cells may include myoblast, muscle satellite cells, myotube cells, myotubes, myofibers, or mature myofibers.

According to yet another aspect, the present application provides a method for reducing serum components in a cell culture medium, the method including bringing the above-described multi-layer film into contact with a cell culture medium containing cells.

In an embodiment, the serum component may be fetal bovine serum.

In an embodiment, the serum component may be less than 10 wt%, more specifically less than 9 wt%, less than 8 wt%, less than 7 wt%, less than 6 wt%, less than 5 wt%, or less than 4 wt% in the cell culture medium.

The lower limit of the serum component content may be 0.001 wt%, 0.01 wt%, or 0.1 wt% in the cell culture medium. Even through the composition of the present application contains the serum component having a content lower than 10 wt%, which is the content of serum components (e.g., fetal bovine serum) contained in a conventional cell culture medium, when the composition contains the above-described multi-layer film of the present application, cell proliferation, more specifically, equal or higher level of proliferation in muscle cell proliferation, is possible.

In an embodiment, the cells may be cells for preparing cultured meat, and specifically, may be animal-derived muscle cells, and the muscle cells may include myoblast, muscle satellite cells, myotube cells, myotubes, myofibers, or mature myofibers.

The description of the multi-layer film in the composition for cell culture, the apparatus for cell culture, the method for preparing cultured meat, and the method for reducing serum components are the same as those described in the multi-layer film, which is another aspect of the present application, and thus is not described in duplicate.

According to still yet another aspect, the present application provides a method for preparing a multi-layer film for cell culture, the method including the following steps of:
(a) preparing a first solution by adjusting the pH of a solution containing a cationic material having an amine group in the side chain thereof to a predetermined range, and preparing a second solution by adjusting the pH of a solution containing an anionic material having a carboxyl group in the side chain thereof to a predetermined range;
(b) alternately contacting the first solution and the second solution on a substrate to alternately stack a layer of cationic material and a layer of anionic material to form a multi-layer structure; and
(c) cross-linking cationic material and anionic material in the multi-layer structure.

In an embodiment, the method may further include, after step (c), (d) a step of incorporating nutrient materials for cell culture in the pores formed by the cross-linking.

In an embodiment, the first solution and the second solution may have a pH of 3-5.

In another embodiment, the method may further include, after step (d), (e) a step of forming a capping layer on the multi-layer structure.

In the method for preparing a multi-layer film for cell culture of the present application, the description of the multi-layer film is the same as that described in the multi-layer film, which is another aspect of the present application, and thus is not described in duplicate.

### ADVANTAGEOUS EFFECTS

According to the multi-layer film of the present application, nutrient materials necessary for cell culture can be delivered stably.

According to the multi-layer film of the present application, the problems of ethics, high costs, and causing diseases can be solved due to the use of nutrient materials which can replace animal-derived serum.

The size, roughness, or porosity of pores inside the multi-layer structure of the multi-layer film of the present application can be easily adjusted, thereby controlling the amount of nutrient materials incorporated, the amount of nutrient materials released, and the speed of nutrient materials released.

By providing the capping layer on the outermost layer of the multi-layer structure of the multi-layer film in the present application, the initial burst release of the incorporated nutrient materials can be controlled, and the stabilization of the nutrient materials can be improved.

However, the effects of the present application are not limited to the above-mentioned effects and other effects not mentioned will be clearly understood from the following description by a person skilled in the art.

### BRIEF DESCRIPTION OF THE DRAWINGS

FIG. 1 shows a process of preparing a multi-layer film based on chitosan (CHI) and carboxymethylcellulose (CMC) by a layer-by-layer assembly (LbL assembly).
FIG. 2 is a graph showing that the thickness of a multi-layer film increases according to the number of stacking CHI/CMC bilayer in the LbL assembly.
FIG. 3 is a graph showing a decrease in the thickness of a multi-layer thin film according to exposure time in a physiological saliva environment.
FIG. 4 shows the result that there is a difference in the released amount and release behavior of C-PC according to the thickness of the multi-layer film.
FIG. 5 is an SEM observation image of a multi-layer film (CHI/CMC)₄₀ sample before cross-linking, a multi-layer film (X-linked) sample after cross-linking, and a multi-layer film (C-PC Loaded) sample after C-PC incorporation.
FIG. 6 is a graph showing the results of analyzing a change in porosity in a thin film after C-PC is incorporated inside a cross-linked multi-layer film.
FIG. 7 is a graph showing the results of analyzing the C-PC release behavior of the film before and after the formation of the capping layer in the cross-linked multi-layer film.
FIG. 8 is a photograph of a C-PC incorporated multi-layer film sample coated on an OHP film.
FIG. 9 shows improvement in the proliferation of C2C12 cells (left graph) by a multi-layer film for C-PC delivery and cell counting results (right graph) by Cell Counting Kit-8 (CCK-8) analysis.

### MODE FOR CARRYING OUT THE INVENTION

Hereinafter, the present application will be described in detail with reference to examples. However, the following examples specifically illustrate the present application, and the contents of the present application are not limited by the following examples.

### Examples

### Example 1: Production of Polysaccharide-based Multi-layer Film

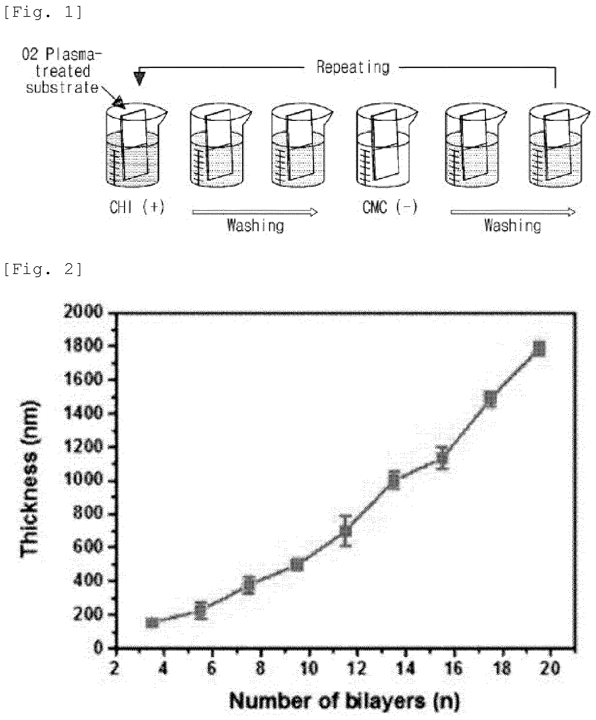

A polysaccharide-based multi-layer film was prepared by a layer-by-layer assembly (LbL assembly) using mutual attraction between materials constituting the layers (FIG. 1).

First, an aqueous solution of chitosan (CHI, Sigma-Aldrich) having a concentration of 1 mg/mL and an aqueous solution of carboxymethylcellulose (CMC, Sigma-Aldrich) having a concentration of 1 mg/mL were prepared, and the pH of the aqueous solutions was adjusted to 4 using 1M NaOH and 1M HCl (**step 1**). Chitosan and carboxymethylcellulose are present in the pH 4 aqueous solution with at least 80% thereof ionized. Subsequently, an oxygen plasma-treated substrate (Si-wafer) was prepared, supported on an aqueous solution of chitosan positively charged for 15 minutes, and then washed twice in tertiary distilled water (**step 2**). Subsequently, the substrate was supported in an aqueous solution of carboxymethylcellulose negatively charged for 10 minutes, and then washed twice in tertiary distilled water (**step 3**).

The CHI/CMC layer was formed by electrostatic attraction between chitosan and carboxymethylcellulose in the above-described steps 2 and 3, and the above-described steps 2 and 3 were repeated to prepare a CHI/CMC multi-layer film. The film prepared by repeating one cycle of the steps 2 and 3 is referred to as a 1 bilayer (BL) film, and as the number of repeating cycles increases, a film having a higher number of BLs was formed (**FIG. 2**). As shown in FIG. 2, an increase in the thickness of the film from 3 BLs to 19 BLs was confirmed, and a relatively linear growth pattern was observed between the number of stacks and the thickness of the multi-layer thin film, and the thickness of 19 BLs was measured to be about 1.8 µm.

### Example 2: Cross-linking and C-PC Incorporation of Polysaccharide-based Multi-layer Film

In order to incorporate C-phycocyanin (C-PC), which is a nutrient material, in the prepared multi-layer film, cross-linking was introduced to the multi-layer film to impart porosity. The substrate coated with the multi-layer film was allowed to stand in a cross-linking solution containing 0.2 M of 1-ethyl-3-(3-dimethylaminopropyl)carbodiimide hydrochloride (EDC) and 5 mM of N-hydroxysuccinimide (NHS) in 0.05 M of a 2-(N-morpholino)ethanesulfonic acid (MES) buffer solution for 30 minutes to perform primary cross-linking and then washed with 1X PBS (**step 4**). Subsequently, the primarily cross-linked multi-layer film was further rinsed with distilled water and left in a 2.5% glutaraldehyde (Sigma-Aldrich) solution for 45 minutes without a drying process to perform secondary cross-linking (**step 5**). After the cross-linked multi-layer film was added to 2 mg/mL of an aqueous C-PC (Sigma-Aldrich) solution, incubation was performed in a dark environment for 12 hours to induce incorporation of C-PC into the porous multi-layer film (**step 6**).

### Example 3: Formation of Capping Layer of Polysaccharide-based Multi-layer Film

After the incorporation of C-PC, in order to prevent the initial burst release of C-PC, an agarose solution (0.25 w/v%) was applied on the surface of the multi-layer film to form a capping layer (**step 7**).

### Example 4: Analysis of Multi-layer Film Characteristics

An analysis of the multi-layer characteristics was performed as follows: The change in mass per unit area was measured by measuring changes in frequency of a quartz crystal microbalance (QCM) device in the process of preparing the coating using an electrode for QCM as a coating substrate by means of the QCM, and the total mass of each material forming the multi-layer film was calculated from the measured change in mass per unit area. The thickness of the multi-layer film was measured using a profilometer, and the porosity, pore size, and volume of the multi-layer film were analyzed using a porosimeter. SEM was used to observe the roughness and porous fine shape of the multi-layer film. The release analysis of nutrient materials was performed by detecting C-PC released from the multi-layer film with photoluminescence spectroscopy (PL) to quantify the release amount over time and analyze the release behavior. C-PC released from the multi-layer film was detected through a micro reader, the release amount over time was quantified and the release behavior was analyzed.

### 1. Measurement of Effect of Inhibiting Decomposition of Multi-Layer Film by Cross-linking

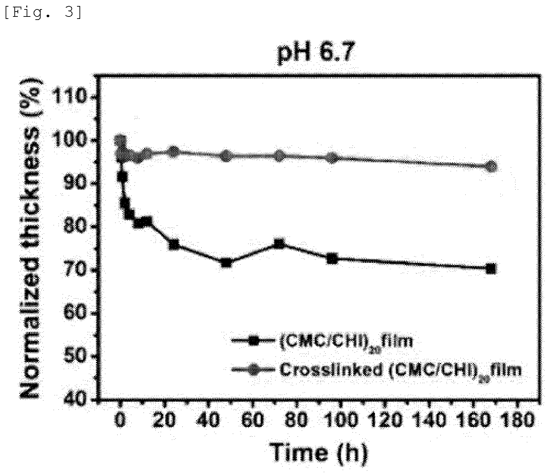

The effect of inhibiting the decomposition of the multi-layer film by cross-linking in the multi-layer film was confirmed by measuring the thickness reduction rate. In the physiological artificial saliva environment, the degree of decomposition of the multi-layer film which was not subjected to the cross-linking was 30% or less, but the degree of decomposition of the multi-layer film which was subjected to the cross-linking was 10% or less, and thus the decomposition of the multi-layer film was reduced by the cross-linking (FIG. 3). This may mean that the effect of the multi-layer film may be maintained for a long period of time, particularly during the process of preparing cultured meat.

### 2. Measurement of Amount of C-PC Released According to Thickness of Multi-Layer Film

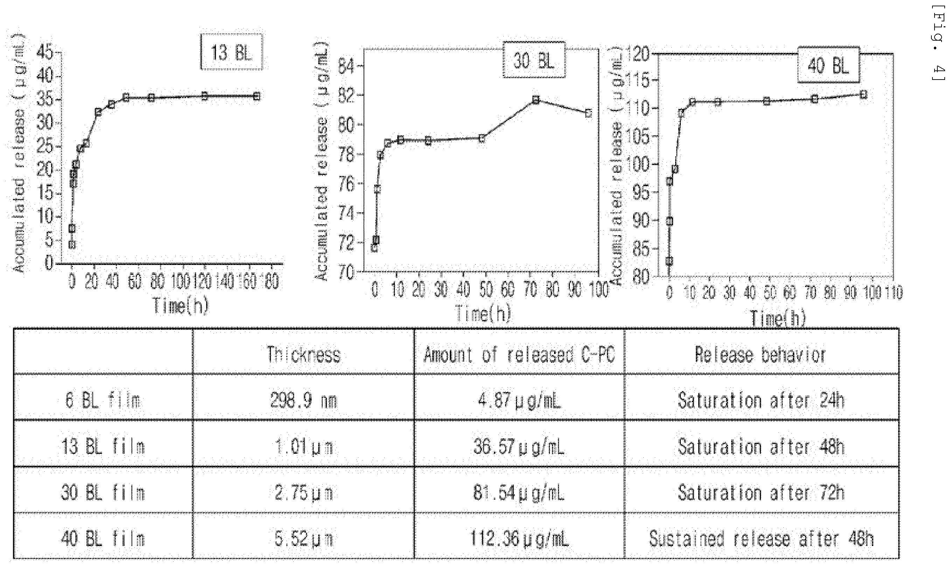

As the thickness of the multi-layer thin film increases, the portion in which C-PC can be incorporated not only increases, but also the formation of small-sized pores in the multi-layer thin film may be promoted by cross-linking, thereby increasing the final amount of C-PC released. Since the increase in the film thickness promotes the formation of small pores in the film, the rate of increase in release was measured to be higher than the rate of increase in thickness. There was a significant difference in the tendency of C-PC released according to a precursor layer/core layer/out-layer of the multi-layer film. That is, as a result of observing the release behavior of C-PC, a burst release due to the diffusion of C-PC from the surface was initially shown, followed by the release due to the C-PC diffusion from the small pores inside the film, which is smaller than the pore size of the film surface. At least 100 ug/mL of C-PC was released from the 40 BL film, and this release amount was identified as the most effective concentration for long-term proliferation and differentiation of C2C12 cells (**FIG. 4**).

### 3. Shape Analysis of Cross-linked Polysaccharide-Based Multi-Layer Film by C-PC Incorporation

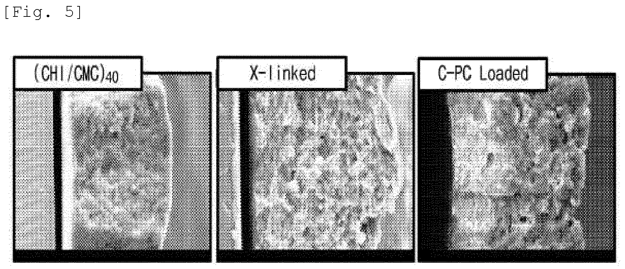

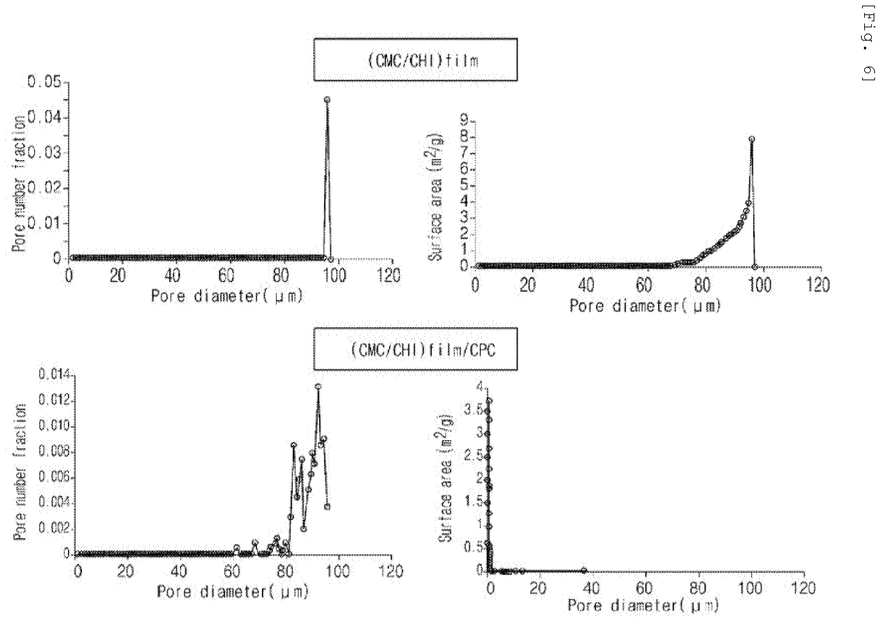

Due to the chain reassembly by the cross-linking, the internal shape of the multi-layer film has an increase in porosity and exhibits rough characteristics, and thus the area in which C-PC can be incorporated inside the multi-layer film is increased, thereby increasing the loading amount. After loading C-PC on the cross-linked multi-layer film, it was observed that pores inside the film were covered by C-PC (FIG. 5). The cross-linked (CMC/CHI)₄₀ film has an apparent pore structure and has a porosity of about 18% with respect to the whole, and the total porosity was reduced to about 5.8% after loading C-PC into the pores (**FIGS. 6** **and Table 1**). A sharp decrease in the intra-particle porosity (%) indicates that C-PC has been incorporated up to the inside of the film.

**[Table 1]**

| | Total interparticle porosity (%) | Total intraparticle porosity (%) | Total porosity (%) |
|---|---|---|---|
| (CMC/CHI) film | 8.6018 | 8.4423 | 18.0442 |
| (CMC/CHI) film/CPC | 5.5061 | 1.3827 | 5.887 |

### 4. Measurement of Amount of C-PC Released According to Preparation of Capping Layer on Surface of Multi-Layer Thin-Film.

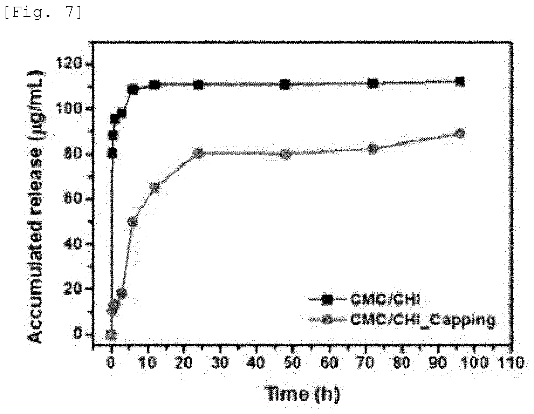

It can be confirmed that the burst release of C-PC was inhibited by the capping layer, and continuous release of C-PC occurred until 96 hours (**FIG. 7**). Therefore, it has been suggested that a method of physically inhibiting the release using the capping layer may be effective in order to prevent the burst release that occurs initially.

### 5. Evaluation of Cell Proliferation of C-PC Incorporated Cross-linked Polysaccharide-Based Multi-layer Film

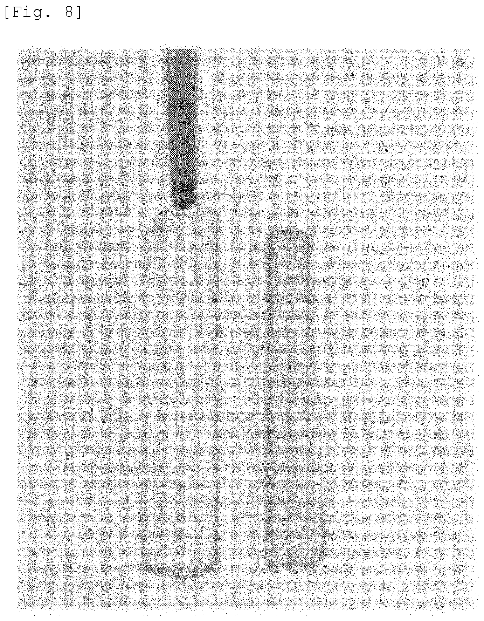

Cell proliferation effects were evaluated by applying the C-PC incorporated multi-layer film to cell culture. The C-PC incorporated multi-layer film sample was prepared according to the method described in Example 1, by preparing a 40 BL multi-layer film using an OHP film as a substrate, and then incorporating C-PC. It was observed that the color of the film was also blue due to the incorporation of the blue-colored C-PC (**FIG. 8**).

In order to confirm the effect of enhancing the proliferation of C2C12 myoblasts by the multi-layer film for C-PC delivery, C2C12 cells were cultured for 7 days in control groups and an experimental groups, respectively. C2C12 myoblasts (Passage 6) was seeded into each well of a 24 well-plate at 3.5 × 10³ each. The culture was performed using a culture medium in which 1% penicillin/streptomycin antibiotic (Gibco^{®} Life Technologies) and 10% or 5% FBS (WELGENE) are contained in Dulbecco's Modified Eagle Medium (DMEM, Gibco^{®} Life Technologies).

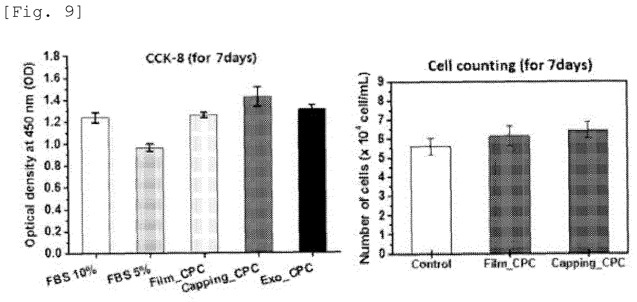

Control group 1 was cultured using a culture medium (FBS 10%) containing 10% FBS, Control group 2 was cultured using a culture medium (FBS 5%) containing 5% FBS, Experimental group 1 was cultured using a culture medium (Film_CPC) prepared by adding a multi-layer film, in which C-PC was incorporated, to a culture medium containing 5% FBS, Experimental group 2 was cultured using a culture medium (Capping_CPC) prepared by adding a multi-layer film, in which C-PC was incorporated and to which an agarose capping layer was applied, to a culture medium containing 5% FBS, and Experimental group 3 was cultured using a culture medium (Exo_CPC) in which C-PC was added to a 5% FBS culture medium at a concentration of 100 µg/mL. As a result of the experiments, the Film_CPC group showed a cell proliferation degree that was almost similar to that of the FBS 10% group, and it was observed that the Capping_CPC group to which the agarose capping layer was applied had a cell proliferation degree higher than the FBS 10% group (**FIG. 9**). Through this, it may be confirmed that FBS can be reduced by the multi-layer film of the present application. It is thought that these resulted from that the agarose, which is a saccharide component, improves the thermal stability of C-PC and at the same time inhibits the burst release from the multi-layer film.

Although the representative embodiments of the present application have been exemplarily described, the scope of the present application is not limited to the specific embodiments as described above, and a person skilled in the art can change the present application within the scope described in the claims of the present application.

## Claims

1. A multi-layer film for cell culture comprising:
(a) a substrate; and
(b) a multi-layer structure which is formed by alternately stacking, on the substrate, a cationic material having an amine group in the side chain thereof, and an anionic material having a carboxyl group in the side chain thereof, and contains pores, and in which the cationic material and the anionic material are cross-linked to each other.

2. The multi-layer film of claim 1, further comprising nutrient materials for cell culture which are incorporated into the pores of the multi-layer structure.

3. The multi-layer film of claim 1, wherein the cationic material having an amine group in the side chain thereof is one selected from the group consisting of chitosan, gelatin, collagen, fibrinogen, silk fibroin, casein, elastin, laminin, fibronectin, polydopamine, polyethyleneimine, poly-l-lysine, poly(vinylamine)hydrochloride, and poly(amino acid).

4. The multi-layer film of claim 1, wherein the anionic material having a carboxyl group in the side chain thereof is one selected from the group consisting of hyaluronic acid, poly-l-lactic acid, alginic acid, dextran sulfate, lignin, tannic acid, chondroitin sulfate, cellulose-based polymers, fucoidan, and heparin.

5. The multi-layer film of claim 1, further comprising a capping layer formed on the multi-layer structure.

6. The multi-layer film of claim 5, wherein the capping layer is prepared by polysaccarides.

7. The multi-layer film of claim 6, wherein the polysaccarides is selected from the group consisting of agarose, carageenan, fucoidan, laminaran, cellulose, hemicellulose, and lignan.

8. The multi-layer film of claim 2, wherein the nutrient material for cell culture is C-phycocyanin; growth factors selected from the group consisting of IGF-1, BMP-2, and FGF-1; amino acids; or ascorbic acid.

9. The multi-layer film of claim 1, wherein the multi-layer film has a thickness of 0.1-10 µm.

10. The multi-layer film of claim 1, wherein the multi-layer structure has a bilayer (BL), which is a stack unit comprising a layer of cationic material and a layer of anionic material, and the BL is in the range of 10-70 BLs.

11. The multi-layer film of claim 1, wherein the multi-layer structure has a porosity of 10-26%.

12. The multi-layer film of claim 1, wherein the multi-layer structure has a degree of cross-linking of 50-80%.

13. A composition for cell culture comprising:
(i) the multi-layer film of any one of claims 1 to 12; and
(ii) serum components.

14. The composition of claim 13, wherein the serum component content is less than 10 wt% in the composition for cell culture.

15. An apparatus for cell culture comprising the multi-layer film of any one of claims 1 to 12.

16. A method for preparing cultured meat comprising bringing the multi-layer film of any one of claims 1 to 12 into contact with a cell medium containing cells.

17. The method of claim 16, wherein the cells are animal-derived muscle cells.

18. A method for reducing serum components in a cell culture medium, the method comprising bringing the multi-layer film of any one of claims 1 to 12 into contact with the cell culture medium containing cells.

19. A method for preparing a multi-layer film for cell culture comprising the steps of:
(a) preparing a first solution by adjusting the pH of a solution containing a cationic material having an amine group in the side chain thereof to a predetermined range, and preparing a second solution by adjusting the pH of a solution containing an anionic material having a carboxyl group in the side chain thereof to a predetermined range;
(b) alternately contacting the first solution and the second solution on a substrate to alternately stack a layer of cationic material and a layer of anionic material to form a multi-layer structure; and
(c) cross-linking cationic material and anionic material in the multi-layer structure.

20. The method of claim 19, further comprising, after step (c), (d) a step of incorporating nutrient materials for cell culture in the pores formed by the cross-linking.

21. The method of claim 19, wherein the first solution and the second solution have a pH of 3-5.

22. The method of claim 20, further comprising, after step (d), (e) a step of forming a capping layer on the multi-layer structure.
